(19) Europäisches Patentamt
European Patent Office
Office européen des brevets

(11) Publication number: 0 028 305
B1

# (12) EUROPEAN PATENT SPECIFICATION

(45) Date of publication of patent specification: 01.02.84

(21) Application number: 80105463.6

(22) Date of filing: 12.09.80

(51) Int. Cl.³: C 07 C 79/34, C 07 C 41/16, C 07 C 43/225, C 07 C 121/52, C 07 C 63/04, C 07 C 51/353, C 07 C 25/08, C 07 C 29/136, C 07 C 33/46, C 07 C 45/62, C 07 C 49/233

## (54) Diaryl compounds and pharmaceutical formulations containing them.

(30) Priority: 13.09.79 GB 7931838

(43) Date of publication of application: 13.05.81 Bulletin 81/19

(45) Publication of the grant of the patent: 01.02.84 Bulletin 84/5

(84) Designated Contracting States: CH DE FR GB IT LI NL SE

(56) References cited:
GB - A - 1 046 380
US - A - 2 817 622

CHEMICAL ABSTRACTS, vol. 74, 1971 page 475, abstract no. 99648e Columbus, Ohio, US

(73) Proprietor: THE WELLCOME FOUNDATION LIMITED
183-193 Euston Road
London NW1 2BP (GB)

(72) Inventor: Hodson, Harold Francis
69 Whitecroft Way Park Langley
Beckenham Kent (GB)
Inventor: Selway, John William Talbot
Lowlands Farm Sand Lane Frittenden
Cranbrook Kent (GB)
Inventor: Leaver, Catherine Mary
1 Renton Drive
Orpington Kent (GB)

(74) Representative: Berg, Wilhelm, Dr. et al,
Dr. Berg, Dipl.-Ing. Stapf, Dipl.-Ing. Schwabe, Dr. Dr. Sandmair Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 028 305 B1

Courier Press, Leamington Spa, England.

# Diaryl compounds and pharmaceutical formulations containing them

The present invention relates to certain diaryl compounds as medicaments. In particular such compounds are antiviral agents, and are especially suitable for the prevention and treatment of rhinoviral infections. The invention also relates to pharmaceutical formulations containing them.

In the majority of instances, the disease known as the "common cold" is caused by rhinoviral infections, although "colds" may also be caused by infection of the upper respiratory tract by e.g. corona and enteroviruses. Mankind throughout the world is prone to rhinoviral infections, which are a major cause of absence from work through illness. The prevention and treatment of such diseases is thus of great economic importance.

Once infected by a rhinovirus, an individual retains immunity to that serotype, which may be enhanced by continual reinfection if the serotype is prevalent in the community. There is however, no cross-immunity between serotypes and thus a cold is usually experienced by an individual whenever a new rhinovirus serotype is encountered, on average about two or three times a year. Immunisation against rhinovirus is not practicable because there are about 120 known serotypes of rhinovirus and a vaccine against all these would overload the vaccinee's immune system. It would therefore appear that chemotherapy is the only suitable method for preventing or treating rhinoviral infections. Much research effort has been expended in recent years but no effective chemotherapeutic agent has yet emerged.

It has now been found that certain biphenyl compounds have antiviral activity and are especially active against rhinoviruses and corona viruses.

GB—A—1,046,380 discloses diaryl compounds, and formulations containing such compounds, which are purportedly useful for lowering mammalian blood cholesterol levels. The abstract of Czechoslavakian Patent No 135,295, published as Chemical Abstracts *74* (1971), 99648e, discloses certain phenyl benzyl thioethers which are reported to have been used as intermediates in the synthesis of psychotropic and antihistamine agents. US—A—2,817,622 discloses certain benzyl phenyl ethers allegedly having insecticidal and bacteriocidal activity.

According to the present invention there is provided *N*-3,4-dichlorobenzylaniline, 4-chlorobenzyl 2-nitrophenyl ether, 4-chlorobenzyl 4-ethylphenyl ether, 4-chlorobenzyl 3-*t*-butylphenyl ether, 3,4-dichlorobenzyl 3,4-dichlorophenyl ether, benzyl 3-trifluoromethylphenyl ether, 4-chlorobenzyl 4-carboxyphenyl ether, 3,4,5-trichlorobenzylphenyl ether, 4-chlorobenzyl 3,4-dichlorophenyl ether, or a compound of the formula (I),

$$Ar—X—Y—Z—Ar \qquad (I)$$

characterised in that the groups Ar are the same or different and each is a substituted or unsubstituted phenyl group, each of the substituents, when present, being selected from halo, (lower)alkyl, hydroxy(lower)alkyl, carboxy(lower)alkyl, (lower)alkoxy, amino, (lower)alkylamino, di(lower)alkylamino, acylamino, nitro, cyano, trifluoromethyl, thiocarbamoyl, carbamoyl, carboxyl, (lower)alkoxycarbonyl and hydroxyl;

Z is $—CH_2—$, —CHOH—, or —(CH=O)—; and

X is oxygen, sulphur, $—CH_2—$ or —NH— when Y is $—CH_2$;

or X is $—CH_2—$ when Y is oxygen;

or X—Y together is —CH=CH—;

provided that, when Z is —(C=O)—, X—Y together do not represent $—CH_2—CH_2$ or —CH=CH—;

or, when a compound of formula (I) bears a hydroxyl, amino or carboxyl group, or when X is an imino group a salt or an ester of such a compound as a medicament.

As used herein the expressions "(lower)alkyl" and "(lower)alkoxy" and cognate terms, mean straight or branched alkyl or alkoxy groups respectively having from 1 to 5 carbon atoms; and "acylamino" means an amino group substituted with the residue of a carboxylic acid, in particular a (lower)alkyl, aryl(lower)alkyl or aryl carboxylic acid.

It is preferred that the salts and esters, when provided, be pharmaceutically acceptable; a discussion of the properties and desirability of various salts is given in "pharmaceutical salts" by S. M. Berge *et al*, *J. Pharm. Sci.*, *66,* 1, (1977). Some of the compounds of formula (I) may also exist in various stereoisomeric forms, all of which are encompassed in the present invention. In particular, those compounds having a carbon-carbon double bond in the group XY will exist as geometric isomers, denoted E and Z; and those having an asymmetric carbon atom, i.e. when Z is a CHOH group, will exist in enantiomeric forms.

Of the various classes of compounds embraced by formula (I), those in which X is oxygen, —NH— or $—CH_2—$, or in which X—Y is —CH=CH—, are preferred. Also preferred are those having two atoms in the chain between the phenyl groups and those wherein Z is $—CH_2—$. Compounds in which each phenyl ring bears no more than three substituents, more preferably one or two substituents, and most preferably a single substituent, for example substituents situated at the 3,4 and/or 5 positions of the phenyl ring of a group Ar and especially at the 4 position, are preferred. Other suitable compounds

are substituted at the 3 and 4 positions of the phenyl rings. Particularly preferred substituents are halo, amino, hydroxyl, methyl, ethyl, methoxy ethoxy, hydroxymethyl, cyano and trifluoromethyl groups.

Compounds of formula (I) are more preferred as medicaments when one Ar group is substituted by 3,4-dichloro, 4-carbamoyl, 3,4-methylenedioxy, 4-chloro or is unsubstituted and the other Ar group is substituted by 3,4-dichloro, 4-chloro or is unsubstituted; provided that taken together the two Ar groups have a total of at least two substituents;

X is —NH—, oxygen or $—CH_2—$,
Y is $—CH_2—$,
or X—Y taken together is —CH=CH—; and
Z is —(C=O)—

According to a second aspect of the present invention there is provided a compound selected from:

*N*-3,4-dichlorobenzylaniline, a salt thereof,
3,4-dichlorobenzyl 3,4-dichlorophenyl ether,
4-chlorobenzyl 4-cyanophenyl ether,
4-chlorobenzyl 3,4-dichlorophenyl ether,
4-(4-chlorobenzyloxy)benzamide,
4-chloro-*N*-(3,4-methylenedioxybenzyl)aniline, a salt thereof,

or a compound of the formula (I'),

$$Ar—X—CH_2—(C=O)—Ar \qquad (I')$$

characterised in that each of the groups Ar is a phenyl group; one Ar group is substituted by 3,4-dichloro, 4-carbamoyl, 3,4-methylenedioxy, 4-chloro or is unsubstituted; and the other Ar group is substituted by 3,4-dichloro, 4-chloro or is unsubstituted; provided that taken together the two Ar groups have a total of at least two substituents;

X is —NH— or oxygen;
or, when X is —NH—, a salt of such a compound.

The present invention provides the following preferred compounds:

1-(3,4-dichlorophenyl)-3-phenyl propane,
3-(3,4-dichlorophenyl)-1-phenylprop-2-ene, and
2-(4-chlorophenoxy)-1-(4-chlorophenyl)ethanol.

Compounds of the invention may be produced by a variety of processes which are well known in the art of organic chemistry. In particular, those compounds having oxygen, nitrogen or sulphur as part of the X—Y moiety will be readily produced by condensation reactions. Thus, for instance, benzyl phenyl ethers are formed by condensation of a phenol with a benzylchloride derivative, and benzyl anilines are produced from benzylchloride derivatives by interaction with an aniline.

Those compounds of the invention wherein X—Y is a hydrocarbon moiety are conveniently produced by forming an unsaturated compound and then (if necessary) reducing the double bond. The 1,3-diphenyl propanes and propenes are produced by first making the corresponding chalcone (by Knoevenagel condensation of acetophenone with benzaldehyde or derivatives thereof) and then reducing either the double bond (to form saturated ketones) and/or reducing the ketone function to provide the alcohol or alkene or alkane. A few of these reactions are illustrated in the Examples hereinbelow. Further details can be obtained from standard chemical texts.

While it is possible for the compounds of the invention or, where appropriate, pharmaceutically acceptable salts thereof (hereinafter referred to as the "active compounds") to be administered as the raw chemical it is preferred that the active compound is presented in the form of a pharmaceutical composition.

In a further aspect of the invention there is therefore provided a pharmaceutical formulation comprising the active compound in association with a pharmaceutically acceptable carrier. The carrier must be "acceptable" in the sense of being compatible with the other ingredients of the formulation and not deleterious to the recipient thereof. Such carriers are solid, liquid or gaseous material recommended for the purpose of administering the medicament.

These pharmaceutical compositions may be administered orally or parenterally (including subcutaneous, intramuscular and intravenous injection) or as a suppository or pessary or may be applied topically or as an ophthalmic solution, or they may be inhaled. It is preferred that the compositions are administered orally or inhaled.

For oral administration the pharmaceutical compositions may be presented as a draught in water or in a syrup, in capsules, cachets, boluses or tablets, as an aqueous or oleaginous solution or suspension or in suspension in a syrup, such suspensions optionally including suspending agents or as an oil-in-water or water-in-oil emulsion. Where desirable or necessary flavouring, sweetening, preserving, thickening or emulsifying agents may be included in the formulation.

Tablets may contain the active compound as a powder or granules optionally mixed with binders,

lubricants or inert diluents or surface-active or dispersing agents and may be formed by compression or by moulding in an inert liquid diluent. Such tablets may be optionally scored and/or coated.

Capsules and cachets may contain the active compound alone or in admixture with one or more accessary ingredients. Capsules may also contain the active compound in aqueous or oleaginous solution suspension or emulsion, optionally in association with accessory ingredients.

For administration as a suppository or pessary the active compound may be presented in admixture with a suitable carrier such as cocoa butter and other material commonly used in the art, the formulation conveniently being shaped by moulding.

For administration in discrete dosage forms such as the tablets, capsules, suppositories and pessaries described above, the active compound is preferably presented at 0.1 mg to 100 mg most preferably 1 mg to 10 mg per tablet, capsule, suppository or pessary.

For parenteral administration the active compound may be presented in sterile solutions or suspensions in aqueous or oleaginous vehicles, which may also contain preservatives and materials for rendering the solution or suspension isotonic with the blood of the intended recipient. Such formulations may conveniently be presented in unit-dose or multi-dose sealed containers. For topical administration the composition may be presented in ointments creams lotions, pastes, jellies, sprays, aerosols and bath oils. Ointments and creams may have oleaginous, absorption and colloidal clay bases and may contain thickening agents such as gum tragacanth or sodium alginate and other pharmaceutically acceptable accessory ingredients such as humectants, preservatives, buffers and antioxidants which are useful in such formulations.

For administration as eye drops, the active compound is presented in sterile water, with excipients such as antimicrobical agents and antioxidants, as a dilute solution. For administration orally in liquid form or parenterally or as eye drops the active compound is preferably presented in solution or suspension or emulsion at a concentration of from 0.1 to 10%, more preferably 0.2 to 5% w/v in unit or multi-dose forms. When presented in unit dose form it is preferred that each dose unit contains 0.1 mg to 100 mg, preferably 1 mg to 10 mg, of active compound. For inhalation the active compound may be presented in association with volatile excipients, as a cream, lotion, paste or ointment or as a finely divided dry powder or in solution for inhalation through a nasal spray, atomiser or insufflator.

All the above formulations are produced by processes which comprise bringing into association the active compound and one or more carriers. Such formulations are therefore provided by a process for producing a pharmaceutical formulation of a compound of the invention comprising bringing into association a compound of the invention and a pharmaceutically acceptable carrier therefor.

Compounds of the invention may be administered to human beings and to other mammals to treat or prevent viral diseases, especially rhinoviral infection. The dosage administered obviously depends on the activity of the active compound and also on the speed with which it is absorbed into the body and on other well-known pharmacological considerations, however it is recommended that the active compound is administered at from 2 ug to 10 mg/kg of animal body weight per day, preferably from 25 ug to 1 mg/kg/day and most preferably about 0.1 to 0.3 mg/kg/day. The active compound may be administered once or several times daily to achieve the required daily dose.

The present invention therefore enables the provisions of a method for treating or preventing viral infections comprising the administration to a mammal in an effective dosage of a non-toxic dose of a compound of the invention or a pharmaceutical formulation thereof.

The invention will now be illustrated with reference to the following Examples. Temperatures are given hereunder in degrees Celsius. Pressures are given hereunder in millimeters of mercury ("mmHg"). (1 mm Hg=133.322 Pa).

Example 1

Preparation of N-3,4-dichlorobenzylaniline hydrochloride

A mixture of aniline (0.93 g) and 3,4-dichlorobenzaldehyde (1.75 g) in ethanol (10 ml) was heated on the steam bath for ten minutes and the resultant solution was allowed to cool to room temperature. The crystalline Schiff's base so formed was filtered, washed with ethanol, dried, and a portion (0.46 g) in ethanol (10 ml) was treated with sodium borohydride (0.17 g). The mixture was stirred at room temperature for 1 hr, evaporated *in vacuo* and the residue was partitioned between water and ether. The ether extract was dried over anhydrous sodium sulphate and evaporated to give an oil which was treated with 10*M* hydrochloric acid (0.83 ml) and evaporated to dryness *in vacuo*. The resultant solid was recrystallised from ethanol to give *N*-3,4-dichlorobenzylaniline hydrochloride, m.pt 203°—212°.

Example 2

Preparation of 4-chlorobenzyl 2-nitrophenyl ether

A mixture of 2-nitrophenol (6.95 g,) and 4-chlorobenzylchloride (4.03 g) in ethanol (40 ml) was treated with 10*M*-aqueous sodium hydroxide (5.5 ml,) and heated at reflux on the steam-bath for 2 hour. The reaction mixture was evaporated *in vacuo* to remove the ethanol and the residue was partitioned between water (50 ml) and ether (50 ml). The ether layer was separated and washed 5 times with 2*M* aqueous sodium hydroxide (50, 50, 50, 25, 25 ml) to remove unreacted 2-nitrophenol.

The ethereal solution was dried over anhydrous sodium sulphate and evaporated *in vacuo*. The resulting solid was recrystallised from ethanol to give 4-chlorobenzyl 2-nitrophenyl ether as pale yellow crystals, m.pt 74°—75°.

Example 3 to 11

The following compounds were produced by a method exactly analogous to that used in Example 2.

| Example no. | Compound m.pt | |
|---|---|---|
| 3 | 4-Chlorobenzyl 4-ethylphenyl ether, | 88.5—89.5° |
| 4 | 4-Chlorobenzyl 3-t-butylphenyl ether, | 43.4—44° |
| 5 | 3,4-Dichlorobenzyl 3,4-dichlorophenyl ether, | 99—100° |
| 6 | Benzyl 3-trifluoromethylphenyl ether, | 65—66° |
| 7 | 4-Chlorobenzyl 4-cyanophenyl ether, | 101—102° |
| 8 | 4-Methylbenzyl 4-methylphenyl ether, | 76—78° |
| 9 | 4-Chlorobenzyl 4-carboxyphenyl ether, | 225—229° |
| 10 | 3,4,5-Trichlorobenzyl phenyl ether, | 71—73° |
| 11 | 4-Chlorobenzyl 3,4-dichlorophenyl ether, | 78—80° |

Example 12

Preparation of 1-(3,4-dichlorophenyl)-3-phenylpropane

3-(3,4-Dichlorophenyl)-1-phenylpropan-1-one (1.8 g), hydrazine hydrate (1.2 ml) and powdered potassium hydroxide (0.53 g) in ethane-1,2-diol (13.4 ml) were heated to 210—220° under reflux for $1\frac{1}{2}$ hours. Some (5 ml) of the diol was distilled off from the mixture and heating was continued for a further 8 hours. The mixture was poured into water (100 ml) and extract with ether (3×50 ml). The ethereal extract was dried over anhydrous sodium sulphate, evaporated under reduced pressure and the residual oil was subjected to column chromatography on silica gel (50 g). Elution with petroleum ether b.pt. 60°—80° gave pure 1-(3,4-dichlorophenyl)-3-phenyl propane as a colourless oil.

Example 13

Preparation of 1,3-bis-(4-chlorophenyl)propan-1-ol

Sodium borohydride (0.34 g) was gradually added to a stirred mixture of 1,3-bis-(4-chlorophenyl)propan-1-one (2.5 g) in ethanol (40 ml) and stirring was continued for a further hour at room temperature. Ethanol was removed *in vacuo* and the residue was partitioned between water and ether. The ether extract was separated, dried over anhydrous sodium sulphate and evaporated *in vacuo*. The resulting solid was recrystallised from ethanol to give 1,3-bis-(4-chlorophenyl)propan-1-ol, m.pt. 63°—64°.

Example 14

Preparation of 3-(3,4-dichlorophenyl)-1-phenylpropan-1-ol

3-(3,4-Dichlorophenyl)-1-phenylpropan-1-ol, m.pt. 55°) was prepared by a method exactly analogous to that used in Example 13.

Example 15

Preparation of 3-(3,4-dichlorophenyl)-1-phenylpropene

A mixture of 3-(3,4-dichlorophenyl)-1-phenylpropan-1-ol (0.65 g) and polyphosphoric acid (0.9 ml) was heated to 140° for 3 hours. The cooled mixture was treated with water (50 ml) and extracted three times with ether. The combined extract was dried over anhydrous sodium sulphate, evaporated under reduced pressure and purified by chromatography on a silica gel (50 g) column, eluting with petrol, b.p. 60—80° 3-(3,4-dichlorophenyl)-1-phenylpropene was obtained as a colourless oil homogeneous by t.l.c. on silica-gel eluting with petroleum ether, b.pt. 60—80° as solvent.

Example 16

Preparation of 3-(3,4-dichlorophenyl)-1-phenylprop-2-en-1-ol

A suspension of 3-(3,4-dichlorophenyl)-1-phenylprop-2-en-1-one (2.0 g) in ethanol (60 ml) was treated with sodium borohydride (0.17 g) and the mixture was stirred at room temperature for one hour. Water (200 ml) was then added and the mixture was extracted three times with ether. The combined extract was dried over sodium sulphate and evaporated under reduced pressure. The residue was recrystallised from a mixture of ether and hexane to give 3-(3,4-dichlorophenyl)-1-phenylprop-2-en-1-ol, m.pt. 52°—53°.

Example 17

Preparation of 1,3-bis-(4-chlorophenyl)prop-2-en-1-ol

1,3-Bis-4-(chlorophenyl)prop-2-en-1-ol (m.pt. 68°—69.5°) was prepared by a method exactly analogous to that used in Example 16.

Example 18

Preparation of 2-(4-chlorophenoxy)-1-(4-chlorophenyl)ethanol

A suspension of 1-(4-chlorophenoxy)-2-(4-chlorophenyl)ethanone (2.5 g) in ethanol (50 ml) was treated with sodium borohydride (0.34 g) and stirred at room temperature for 1 hr. The solution was treated with water until a solid was precipitated and the mixture was extracted three times with ether. The combined ether extract was dried over anhydrous sodium sulphate and evaporated *in vacuo.* The residue was recrystallised from a mixture of ethanol and water to give 2-(4-chlorophenoxy)-1-(4-chlorophenyl)ethanol, m.pt 71°.

Example 19

Preparation of 2-(3,4-dichlorophenoxy)-1-phenylethanol

2-(3,4-Dichlorophenyl)-1-phenylethanol, a crystalline solid, (m.pt. 70.5°—71.5°), was prepared by a method exactly analogous to that used in Example 18.

Example 20

Preparation of 4-(4-chlorobenzyloxy)benzenecarbothioamide

A solution of 4-chlorobenzyl 4-cyanophenyl ether (2.43 g) in dry pyridine (90 ml) and triethylamine (12 ml) was treated with a steady stream of gaseous hydrogen sulphide for $1\frac{1}{2}$ hours. The solution was treated with an excess of water and the resultant solid was filtered, washed with water and recrystallised from ethanol to give 4-(4-chlorobenzyloxy)benzenecarbothioamide, m.pt 200°—205°.

Example 21

Preparation of 4-(4-chlorobenzyloxy)benzamide

A mixture of 4-chlorobenzyl 4-cyanobenzyl ether (2.43 g), 10$\underline{M}$ aqueous sodium hydroxide (0.24 ml) and hydrogen peroxide solution (4 ml) in ethanol (30 ml) was stirred at 50° for 3 hours. The mixture was cooled and filtered and the solid was recrystallised from chloroform to give 4-(4-chlorobenzyloxy)benzamide, m.pt. 212°—217°.

Example 22

Preparation of 4-chlorobenzyl 3-hydroxymethylphenyl ether

A solution of 3-(4-chlorobenzyloxy)benzaldehyde (1.23 g) in ethanol (25 ml) was treated with sodium borohydride (0.19 g) and the mixture was stirred at room temperature for 30 minutes. The solution was treated with water until a solid precipitated out. The solid was filtered, washed with a mixture of water and ethanol, dried and recrystallised from ethanol to give 4-chlorobenzyl 3-hydroxymethylphenyl ether, m.pt. 82°.

Example 23

Preparation of 2-(4-chloroanilino)-1-(4-chlorophenyl)ethanol

A suspension of 2-(4-chloroanilino)-1-(4-chlorophenyl)ethanone (1 g) in ethanol (50 ml) was treated with sodium borohydride (0.19 g) and the mixture was stirred at room temperature for one hour during which time the solid dissolved. Most of the ethanol was removed *in vacuo* and the residue was partitioned between water and ether. The ether solution was dried over anhydrous sodium sulphate and evaporated to give material which was purified by column chromatography on silica gel (45 g). The product was eluted with chloroform and recrystallised from a mixture of isopropanol and water to give 2-(4-chloroanilino)-1-(4-chlorophenyl)ethanol, m.pt. 58°—61°.

Example 24

Assay of activity of compounds of the invention

Activity may be detected by the plaque inhibition (PI) test and measured by the plaque reduction (PR) test. Both assays involve the formation of a monolayer cell culture in a petri dish followed by infection with a virus suspension, and then overlaying the culture with nutrient agarose in the form of a gel. This gel ensures that there is no spread of virus throughout the culture and thus areas of localised cell destruction or plaques are formed.

In the plaque inhibition test a filter paper disc which holds 0.01 ml when impregnated with a solution of a compound is placed on top of the agarose gel. The compound may then diffuse throughout the gel so that its greatest concentration will be around the disc and its lowest concentration towards the periphery of the petri dish. The efficacy of the compound may be detected by observing the zone of inhibition of plaque formation.

Detectable activity is measured with the plaque reduction assay. A range of concentrations of compounds of known molarity are incorporated in the nutrient agarose overlay. Plaque suppression is proportional to compound concentration. Plaque numbers are expressed as percentages of a control, and a dose response curve may be drawn. From this curve 50% of the effective dose ($ED_{50}$) may be estimated.

Results

| Compound of example no. | PR($ED_{50}$/uM) |
|---|---|
| 1 | 0.17 |
| 2 | 1.20 |
| 3 | 0.72 |
| 4 | 1.90 (5.0) |
| 5 | 0.47 |
| 6 | 0.66 (1.7) |
| 7 | 0.22 |
| 8 | 0.56 |
| 9 | 1.60 |
| 10 | 1.60 |
| 11 | 0.33 |
| 12 | 0.068 |
| 13 | 1.30 |
| 14 | 0.66 |
| 15 | 0.19 |
| 16 | 1.20 |
| 17 | 1.35 |
| 18 | 1.50 |
| 19 | 2.5 |
| 20 | 0.60 |
| 21 | 0.43 |
| 22 | 0.53 |
| 23 | 3.00 |

Example 25

Intranasal administration—simulation in vitro

Petri dishes were prepared, as for the plaque inhibition test and the confluent sheet of cells was covered with a layer of agarose gel. The compound of Example 12 (1ug) was dissolved in ethanol, and applied to the lids of the petri dishes. When the ethanol had evaporated, leaving the compound spread over the inside of the lids, these were replaced on the petri dishes. Sufficient compound penetrated the agarose layer to cause total inhibition of plaque formation.

Example 26 to 77

The following compositions were prepared according to the techniques known in the art of pharmacy.

Example 26

An inhalant for use in an insufflator was prepared from the following ingredients

| | |
|---|---|
| Compound of Example 12 | 0.6 g |
| isopropylmyristate | 10 g |
| Tween 80 | 0.5 g |
| Span 60 | 0.5 g |
| methyl-*p*-hydroxybenzoic acid | 0.1 g |
| Water | to 100 ml |

Example 27

A suspension for use as nose drops was prepared from the following ingredients

| | |
|---|---|
| Compound of Example 12 | 0.6 g |
| Keltrol | 0.1 g |
| Sodium Chloride | 0.5 g |
| Sodium lauryl sulphate | 0.1 g |
| Methyl-*p*-hydroxybenzoic acid | 0.1 g |
| Water | to 100 ml. |

Example 28

Capsule 1

| | |
|---|---|
| Compound of Example 12 | 6 g |
| Spray-dried lactose | 300 g. |

Gelatin capsules (size 0) were each filled with 500 mg. of the formulation, affording 10 mg. of active ingredient per capsule.

Example 29
Capsule 2

| | |
|---|---|
| Compound of Example 12 | 6 g |
| Spray-dried lactose | 208 g |
| Maize starch | 20.8 g |
| Polyvinylpyrollidine | 5.2 g |

Gelatin capsules (size 1) were each filled with 400 mg. of the formulation, affording 10 mg. of the active ingredient per capsule.

Example 30
Tablet of the compound of Example 12

A tablet formulation containing a mixture of the compound (10 mg), lactose (90 mg), maize starch (10 mg) and magnesium stearate (1 mg) is prepared by wet granulation.

Example 31 to 53

Tablet formulations, each containing one of the flavan derivatives of Examples 1 to 11 and 13 to 23 are prepared by a method exactly analogous to Example 30.

Example 54
Oil formulation of 1-(3,4-dichlorophenyl)-3-phenylpropane (compound of Example 12)

olive oil B.P. 1 g

The compound was dissolved in olive oil for use by oral administration.

Examples 55 to 77

Oil formulations of the compounds of Examples 1 to 11 and 13 to 23 were prepared by a method exactly analogous to that of Example 54.

**Claims**

1. *N*-3,4-Dichlorobenzylaniline, 4-chlorobenzyl 2-nitrophenyl ether, 4-chlorobenzyl 4-ethylphenyl ether, 4-chlorobenzyl 3-*t*-butylphenyl ether, 3,4-dichlorobenzyl 3,4-dichlorophenyl ether, benzyl 3-trifluoromethylphenyl ether, 4-chlorobenzyl 4-carboxyphenyl ether, 3,4,5-trichlorobenzylphenyl ether, 4-chlorobenzyl 3,4-dichlorophenyl ether, or a compound of the formula (I),

$$Ar—X—Y—Z—Ar \qquad (I)$$

characterised in that the groups Ar are the same or different and each is a substituted or unsubstituted phenyl group, each of the substituents, when present, being selected from halo, (lower)alkyl, hydroxy(lower)alkyl, carboxy(lower)alkyl, (lower)alkoxy, amino(lower)alkylamino, di(lower)alkylamino, acylamino, nitro, cyano, trifluoromethyl, thiocarbamoyl, carbamoyl, carboxyl, (lower)alkoxycarbonyl and hydroxyl;

Z is $—CH_2—$, —CHOH—, or —(C=O)—; and

X is oxygen, sulphur, $—CH_2—$ or —NH— when Y is $—CH_2—$;

or X is $—CH_2—$ when Y is oxygen; or

X—Y together is —CH=CH—;

provided that, when Z is —(C=O)—, X—Y together do not represent $—CH_2—CH_2—$ or —CH=CH—; or, when a compound of formula (I) bears a hydroxyl, amino or carboxyl group, or when X is an imino group, a salt or an ester of such a compound as a medicament.

2. A compound as claimed in claim 1 characterised in that at least one Ar group is substituted in the 3- and 4- position or in the 3-, 4- and 5- position.

3. A compound as claimed in claim 1 characterised in that both Ar groups are substituted in the 4-position or both groups are substituted in the 3- and 4-positions.

4. A compound as claimed in claim 1 characterised in that one Ar group is substituted by 3,4-dichloro, 4-carbamoyl, 3,4-methylenedioxy, 4-chloro or is unsubstituted and that the other Ar group is substituted by 3,4-dichloro, 4-chloro or is unsubstituted; provided that taken together the two Ar groups have a total of at least two substituents;

X is —NH—, oxygen or $—CH_2—$,

Y is $—CH_2—$,

or X—Y together is —CH=CH—; and

Z is —(C=O).

5. A compound as claimed in claim 4 characterised in that one Ar group is substituted by 4-Cl and the other Ar group is substituted by 4-Cl or 3,4-dichloro.

6. A compound selected from:
*N*-3,4-dichlorobenzylaniline, a salt thereof,
3,4-dichlorobenzyl 3,4-dichlorophenyl ether,
4-chlorobenzyl 4-cyanophenyl ether,
4-chlorobenzyl 3,4-dichlorophenyl ether,
4-(4-chlorobenzyloxy)benzamide,
4-chloro-N-(3,4-methylenedioxybenzyl)aniline, a salt thereof,
or a compound of the formula (I'),

$$Ar—X—CH_2—(C{=}O)—Ar \quad (I')$$

characterised in that each of the groups Ar is a phenyl group; one Ar group is substituted by 3,4-dichloro, 4-carbamoyl, 3,4-methylenedioxy, 4-chloro or is unsubstituted; and the other Ar group is substituted by 3,4-dichloro, 4-chloro or is unsubstituted; provided that taken together the two Ar groups have a total of at least two substituents;

X is —NH— or oxygen;

or, when X is —NH—, a salt of such a compound.

7. A compound selected from:
1-(3,4-dichlorophenyl-3-phenyl propane,
3-(3,4-dichlorophenyl)-1-phenylprop-2-ene, and
2-(4-chlorophenoxy)-1-(4-chlorophenyl)ethanol.

8. A pharmaceutical formulation characterised in that it comprises a compound as defined in any one of claims 1 to 7 in association with a pharmaceutically acceptable carrier.

9. A formulation as claimed in claim 8 characterised in that it is in the form of an orally ingestible tablet or capsule.

10. A formulation as claimed in claim 8 characterised in that it is in the form of nose drops.

**Revendications**

1. N-3,4-dichlorobenzylaniline, éther de 4-chlorobenzyle et de 2-nitrophényle, éther de 4-chlorobenzyle et de 4-éthylphényle, éther de 4-chlorobenzyle et de 3-t-butylphényle, éther de 3,4-dichlorobenzyle et de 3,4-dichlorophényle, éther de benzyle et de 3-trifluorométhylphényle, éther de 4-chlorobenzyle et de 4-carboxyphényle, éther de 3,4,5-trichlorobenzyle et de phényle, éther de 4-chlorobenzyle et de 3,4-dichlorophényle, ou un composé de formule (I),

$$Ar—X—Y—Z—Ar \quad (I)$$

dans laquelle les groupes Ar sont identiques ou différents et représentent chacun un groupe phényle substitué ou non substitué, chacun des substituants, éventuellement présents, étant choisi entre les radicaux halogéno, (alkyle inférieur), hydroxy(alkyle inférieur), carboxy(alkyle inférieur), alkoxy inférieur, amino(alkyle inférieur)amino, di(alkyle inférieur)amino, acylamino, nitro, cyano, trifluorométhyle, thiocarbamoyle, carbamoyle, carboxyle, (alkoxy inférieur)carbonyle et hydroxyle;
Z est —$CH_2$—, —CHOH—, ou —(C=O)—; et
X est l'oxygène, le soufre, —$CH_2$— ou —NH—
lorsque Y est —$CH_2$—; ou X est —$CH_2$— lorsque
Y est l'oxygène; ou X—Y représentent ensemble —CH=CH—;
à condition que, lorsque Z est —(C=O)—, X—Y ne représentent pas ensemble —$CH_2$—$CH_2$— ou —CH=CH—.
ou, lorqu'un composé de formule (I) porte un groupe hydroxyle, amino ou carboxyle, ou lorsque X est un groupe imino, un sel ou ester de ce composé, à titre de médicament.

2. Composé selon la revendication 1, caractérisé en ce qu'un groupe Ar au moins est substitué en positions 3 et 4 ou en positions 3, 4 et 5.

3. Composé selon la revendication 1, caractérisé en ce que les deux groupes Ar sont substitués en position 4 ou bien les deux groupes sont substitués en positions 3 et 4.

4. Composé selon la revendication 1, caractérisé en ce qu'un groupe Ar est substitué par un groupe 3,4-dichloro, 4-carbamoyle, 3,4-méthylènedioxy, 4-chloro ou est non substitué et en ce que l'autre groupe Ar est substitué par un groupe 3,4-dichloro, 4-chloro ou est non substitué; à condition que, considérés ensemble, les deux groupes Ar présentent un total d'au moins deux substituants;
X est —NH—, l'oxygène ou —$CH_2$—,
Y est —$CH_2$—,
ou X—Y représentent ensemble —CH=CH—; et
Z est —(C=O)—.

5. Composé selon la revendication 4, caractérisé en ce qu'un groupe Ar est substitué par un groupe 4-Cl et l'autre groupe Ar est substitué par un groupe 4-Cl ou 3,4-dichloro.

6. Composé choisi parmi les suivants:
N-3,4-dichlorobenzylaniline, ou un de ses sels,
éther de 3,4-dichlorobenzyle et de 3,4-dichlorophényle,
éther de 4-chlorobenzyle et de 4-cyanophényle,
éther de 4-chlorobenzyle et de 3,4-dichlorophényle,
4-(4-chlorobenzyloxy)benzamide,
4-chloro-N-(3,4-méthylènedioxybenzyl)aniline, ou un de ses sels,
ou un composé de formule (I'),

$$Ar—X—CH_2—(C{=}O)—Ar \qquad (I')$$

dans laquelle chacun des groupes Ar est un groupe phényle; un groupe Ar est substitué par un groupe 3,4-dichloro, 4-carbamoyle, 3,4-méthylènedioxy, 4-chloro ou est non substitué; et l'autre groupe Ar est substitué par un groupe 3,4-dichloro, 4-chloro ou est non substitué; à condition que, considérés ensemble, les deux groupes Ar présentent un total d'au moins deux substituants;

X est —NH— ou l'oxygène;

ou, lorsque X est —NH—, un sel de ce composé.

7. Composé choisi parmi les suivants:
1-(3,4-dichlorophényl)-3-phénylpropane,
3-(3,4-dichlorophényl)-1-phénylprop-2-ène, et
2-(4-chlorophénoxy)-1-(4-chlorophényl)éthanol.

8. Formulation pharmaceutique, caractérisé en ce qu'elle contient un composé tel que défini selon l'une quelconque des revendications 1 à 7 en association avec un support pharmaceutiquement acceptable.

9. Formulation selon la revendication 8, caractérisée en ce qu'elle se présente sous la forme d'un comprimé ou d'une capsule pouvant être ingérés oralement.

10. Formulation selon la revendication 8, caractérisée en ce qu'elle se présente sous la forme de gouttes nasales.

**Patentansprüche**

1. N-3,4-Dichlorobenzylanilin, 4-Chlorobenzyl-2-nitrophenyl-äther, 4-Chlorobenzyl-4-ethylphenyl-äther, 4-Chlorobenzyl-3-t-butylphenyl-äther, 3,4-Dichlorobenzyl-3,4-dichlorophenyl-äther, Benzyl-3-trifluoromethylphenyl-äther, 4-Chlorobenzyl-4-carboxyphenyl-äther, 3,4,5-Trichlorobenzylphenyl-äther, 4-Chlorobenzyl-3,4-dichlorophenyl-äther oder eine Verbindung der Formel (I)

$$Ar—X—Y—Z—Ar \qquad (I)$$

dadurch gekennzeichnet, daß die Gruppen Ar gleich oder voneinander verschieden sind und jeweils eine substituierte oder unsubstituierte Phenylgruppe bedeuten, wobei jeder der Substituenten, falls vorhanden, ausgewählt wird aus Halogen, (Niedrig)alkyl, Hydroxy(niedrig)alkyl, Carboxy(niedrig)alkyl, (Niedrig)alkoxy, Amino(niedrig)alkylamino, Di(niedrig)alkylamino, Acylamino, Nitro, Cyano, Trifluoromethyl, Thiocarbamoyl, Carbamoyl, Carboxyl, (Niedrig)alkoxycarbonyl und Hydroxyl;
Z $—CH_2—$, —CHOH— oder —(C=O)— und
X Sauerstoff, Schwefel, $—CH_2—$ oder —NH—, wenn Y $—CH_2—$ ist, oder
X $—CH_2—$, wenn Y Sauerstoff ist, oder
X—Y gemeinsam —CH=CH— bedeuten;
mit der Maßgabe, daß dann, wenn Z —(C=O)— ist, X—Y gemeinsam nicht $—CH_2—CH_2—$ oder —CH=CH— bedeuten; oder wenn eine Verbindung der Formel (I) eine Hydroxyl-, Aminooder Carboxylgruppe trägt oder, wenn X eine Iminogruppe, ein Salz oder ein Ester einer solchen Verbindung als Arzneimittel.

2. Verbindung nach Anspruch 1, dadurch gekennzeichnet, daß mindestens eine Ar-Gruppe in der 3- und 4-Position oder in der 3-, 4- und 5-Position substituiert ist.

3. Verbindung nach Anspruch 1, dadurch gekennzeichnet, daß beide Ar-Gruppen in der 4-Position substituiert sind oder beide Gruppen in den 3- und 4-Positionen substituiert sind.

4. Verbindung nach Anspruch 1, dadurch gekennzeichnet, daß eine Ar-Gruppe substituiert ist durch 3,4-Dichloro, 4-Carbamoyl, 3,4-Methylendioxy, 4-Chloro oder unsubstituiert ist, und daß die andere Ar-Gruppe substituiert ist durch 3,4-Dichloro, 4-Chloro oder unsubstituiert ist, mit der Maßgabe, daß die beiden Ar-Gruppen zusammengenommen insgesamt mindestens zwei Substituenten aufweisen;

X —NH—, Sauerstoff oder —$CH_2$—,
Y —$CH_2$— oder
X—Y gemeinsam —CH=CH— und
Z —(C=O) bedeuten.

5. Verbindung nach Anspruch 4, dadurch gekennzeichnet, daß eine Ar-Gruppe substituiert ist durch 4-Cl und daß die andere Ar-Gruppe substituiert ist durch 4-Cl oder 3,4-Dichloro.

6. Verbindung, ausgewählt aus:
N-3,4-Dichlorobenzylanilin, ein Salz davon,
3,4-Dichlorobenzyl-3,4-dichlorophenyl-äther,
4-Chlorobenzyl-4-cyanophenyl-äther,
4-Chlorobenzyl-3,4-dichlorophenyl-äther,
4-(4-Chlorobenzyloxy)benzamid,
4-Chloro-N-(3,4-methylendioxybenzyl)anilin, ein Salz davon,
oder eine Verbindung der Formel (I′)

$$Ar—X—CH_2—(C{=}O)—Ar \qquad (I')$$

dadurch gekennzeichnet, daß jede der Gruppen Ar eine Phenylgruppe ist, daß eine Ar-Gruppe substituiert ist durch 3,4-Dichloro, 4-Carbamoyl, 3,4-Methylendioxy, 4-Chloro oder unsubstituiert ist, und daß die andere Ar-Gruppe substituiert ist durch 3,4-Dichloro, 4-Chloro oder unsubstituiert ist; mit der Maßgabe, daß die beiden Ar-Gruppen zusammengenommen insgesamt mindestens zwei Substituenten aufweisen,

X —NH— oder Sauerstoff bedeutet oder,

wenn X —NH— ist, ein Salz einer solchen Verbindung.

7. Verbindung, ausgewählt aus:
1-(3,4-Dichlorophenyl-3-phenyl-propan,
3-(3,4-Dichlorophenyl)-1-phenylprop-2-en und
2-(4-Chlorophenoxy)-1-(4-chlorophenyl)ethanol.

8. Pharmazeutische Zubereitung, dadurch gekennzeichnet, daß sie eine Verbindung, wie in einem der Ansprüche 1 bis 7 definiert, in Assoziation mit einem pharmazeutisch verträglichen Träger enthält.

9. Zubereitung nach Anspruch 8, dadurch gekennzeichnet, daß sie in Form einer oral einnehmbaren Tablette oder Kapsel vorliegt.

10. Zubereitung nach Anspruch 8, dadurch gekennzeichnet, daß sie in Form von Nasentropfen vorliegt.